# EUROPEAN PATENT SPECIFICATION

(11) **EP 0 752 406 B1**
(45) Date of publication and mention of the grant of the patent: **06.02.2002**
(21) Application number: 96302734.7
(22) Date of filing: 18.04.1996
(51) Int. Cl.: C07C 51/12, C07C 53/08

(54) **Process for the production of acetic acid by carbonylation**
Verfahren zur Herstellung von Essigsäure durch Carbonylierung
Procédé de préparation d'acide acétique par carbonylation

(30) Priority: 21.06.1995 GB 9512606; 19.07.1995 GB 9514745; 06.10.1995 GB 9520441; 23.11.1995 GB 9524037
(43) Date of publication of application: 08.01.1997
(73) Proprietor: BP Chemicals Limited, London EC2M 7BA (GB)
(72) Inventor: Baker, Michael James, Res. Centre Warrensville, Warrensville, Ohio 44128-2837 (US); Giles, Martin Francis, London, EC2M 7BA (GB); Garland, Carl Sherman, Silver Springs, Maryland 20901 (US); Muskett, Michael James, Hull, HU12 8DS (GB); Rafeletos, Georgios, Canterbury, Kent CT2 7SR (GB); Smith, Stephen, James, Hull HU12 8DS (GB); Sunley, John, Glenn, Hull HU12 8DS (GB); Watt, Robert, John, Hull HU12 8DS (GB); Williams, Bruce Leo, Hull HU12 8DS (GB)
(74) Representative: Perkins, Nicholas David

(56) References cited:
- EP-A- 0 161 874
- EP-A- 0 618 184
- EP-A- 0 643 034

## Description

The present invention relates to a process for the production of acetic acid and in particular, to a process for the production of acetic acid by carbonylation in the presence of an iridium catalyst and methyl iodide co-catalyst.

Preparation of acetic acid by rhodium-catalysed carbonylation processes is known and is described, for example in EP-A-161 874 which relates to the use of iodide salts to stabilise the rhodium catalyst at low water concentrations.

Preparation of carboxylic acids by iridium-catalysed carbonylation processes is known and is described, for example in GB-A-1234121, US-A-3772380, DE-A-1767150, EP-A-0616997, EP-A-0618184, EP-A-0618183 and EP-A-0657386.

GB-A-1234121, US-A-3772380 and DE-A-1767150 describe iridium-catalysed carbonylation processes which do not use promoters as in the present invention.

EP-A-0618184 describes a carbonylation process for the production of carboxylic acids and/or their esters in the presence of an iridium catalyst. The reaction composition is characterised as comprising between 0 exclusive and 10 % water; between 0 exclusive and 10 % halogenated co-catalyst; between 2 and 40 % ester and carboxylic acid solvent. EP-A-0618184 does not describe the use of a promoter.

EP-A-0618183 describes a carbonylation process for the preparation of carboxylic acids, for example acetic acid, in the presence of iridium and rhodium compounds.

EP-A-0657386 describes a process for the preparation of an iridium catalyst solution and its use in a carbonylation reaction for the preparation of acetic acid.

EP-A-0643034 describes a process for the carbonylation of methanol and/or a reactive derivative thereof in the presence of acetic acid, an iridium catalyst, methyl iodide, at least a finite concentration of water, methyl acetate and a promoter selected from ruthenium and osmium. Batch and continuous experiments are described therein. In the continuous experiments the water concentration is as low as 6.8 % by weight.

There remains a need for an improved iridium-catalysed carbonylation process.

Thus, according to the present invention there is provided a process for the production of acetic acid comprising (1) continuously feeding methanol and/or a reactive derivative thereof and carbon monoxide to a carbonylation reactor which contains a liquid reaction composition comprising an iridium carbonylation catalyst, methyl iodide co-catalyst, a finite concentration of water, acetic acid, methyl acetate and at least one promoter; (2) contacting the methanol and/or reactive derivative thereof with the carbon monoxide in the liquid reaction composition to produce acetic acid; and (3) recovering acetic acid from the liquid reaction composition characterised in that there is continuously maintained in the liquid reaction composition throughout the course of the reaction (a) water at a concentration of 1 to 6.5 % by weight, (b) methyl acetate at a concentration in the range 5 to 30 % by weight and (c) methyl iodide at a concentration in the range 5 to 16 % by weight and further in that the water concentration is that which gives the maximum carbonylation rate at the defined methyl acetate and methyl iodide concentrations.

The present invention solves the technical problem defined above by continuously maintaining a liquid reaction composition having defined water, methyl iodide and methyl acetate concentrations. This provides several technical advantages.

Thus in the present invention the rate of the carbonylation reaction increases as the water concentration in the liquid reaction composition is reduced from a concentration of greater than 6.5% by weight, passes through a maximum at a water concentration of no greater than 6.5 % by weight and then declines as very low water concentrations are approached. Therefore, the rate of carbonylation reaction in the process of the present invention will be generally greater than that at a water concentration of greater than 6.5 % by weight (all other parameters being equal, except the variation in the water concentration being off-set by a variation in the acetic acid concentration). The water concentration at which the carbonylation rate is a maximum increases as the concentration of methyl acetate in the liquid reaction composition is increased. It is believed that the water concentration at which the carbonylation rate is a maximum decreases as the concentration of methyl iodide in the liquid reaction composition is increased.

Also, it has been found that the promotional effect of a promoter according to the present invention, such as ruthenium, increases as the water concentration is reduced according to the present invention. As noted below, the beneficial effect of a promoter according to the present invention, such as ruthenium has been found to be greatest at the maximum carbonylation rate in the relationship between carbonylation rate and water concentration. That is, the beneficial effect of a promoter according to the present invention, such as ruthenium, has been found to be greatest at the water concentration which gives the maximum carbonylation rate at any defined methyl acetate and methyl iodide concentrations. In the process of the present invention, this water concentration is no greater than 6.5 % by weight.

Also, in the process of the present invention, by operating at a water concentration of no greater than 6.5 % by weight, recovery of acetic acid from the reaction composition withdrawn from the carbonylation reactor is facilitated because the amount of water which has to be separated from the acetic acid is reduced; separation of water from the acetic acid is an energy-intensive part of the recovery process and reduced water concentration results in reduced processing difficulty and/or costs.

The increased carbonylation rate at the low water concentration of the present invention may allow operation at a reduced iridium catalyst concentration whilst maintaining the rate of carbonylation. This has benefits of reduced production rate of by-products such as propionic acid.

Water may be formed in situ in the liquid reaction composition, for example, by the esterification reaction between methanol reactant and acetic acid product. Small amounts of water may also be produced by hydrogenation of methanol to produce methane and water. Water may be introduced to the carbonylation reactor together with or separately from other components of the liquid reaction composition. Water may be separated from other components of reaction composition withdrawn from the reactor and may be recycled in controlled amounts to maintain the required concentration of water in the liquid reaction composition. The water concentration in the liquid reaction composition is from 1 to 6.5% by weight and preferably no greater than 6 % by weight.

In the process of the present invention, suitable reactive derivatives of methanol include methyl acetate, dimethyl ether and methyl iodide. A mixture of methanol and reactive derivatives thereof may be used as reactants in the process of the present invention. Preferably, methanol and/or methyl acetate are used as reactants. At least some of the methanol and/or reactive derivative thereof will be converted to, and hence present as, methyl acetate in the liquid reaction composition by reaction with acetic acid product or solvent. In the process of the present invention the concentration of methyl acetate in the liquid reaction composition is in the range 5 to 30 % by weight, more preferably 5 to 25% by weight It has been found that as the methyl acetate concentration is increased, the rate of carbonylation reaction increases and the selectivity to by-products such as propionic acid and carbon dioxide decreases. However, as the concentration of methyl acetate is increased the amount that has to be recycled to the carbonylation reactor from the acetic acid recovery stage increases. Also, too high a methyl acetate concentration may adversely affect phase separation of aqueous and methyl iodide phases during the acetic acid recovery stage. Also, too high a methyl acetate concentration may adversely affect the carbonylation reaction rate by reducing the partial pressure of carbon monoxide at a defined total pressure in the carbonylation reactor.

In the process of the present invention, the concentration of methyl iodide co-catalyst in the liquid reaction composition is in the range 5 to 16 % by weight. As the methyl iodide co-catalyst concentration is increased, the rate of production of by-products such as propionic acid, carbon dioxide and methane is reduced. The increase in rate of carbonylation brought about by increasing methyl iodide concentration is greater at lower water concentrations than at higher water concentrations. Also, as the concentration of methyl iodide is increased, phase separation of aqueous and methyl iodide phases in the acetic acid recovery stage may be facilitated. However, as the concentration of methyl iodide is increased, the partial pressure of carbon monoxide may be adversely reduced at a defined total pressure in the carbonylation reactor.

In the process of the present invention, the iridium carbonylation catalyst is preferably present in the liquid reaction composition at a concentration in the range 400 to 5000 ppm measured as iridium, more preferably in the range 500 to 3000 ppm measured as iridium, yet more preferably in the range 700 to 3000 ppm measured as iridium. In the process of the present invention, the rate of the carbonylation reaction increases as the concentration of iridium is increased.

The iridium catalyst in the liquid reaction composition may comprise any iridium-containing compound which is soluble in the liquid reaction composition. The iridium catalyst may be added to the liquid reaction composition for the carbonylation reaction in any suitable form which dissolves in the liquid reaction composition or is convertible to a soluble form. Examples of suitable iridium-containing compounds which may be added to the liquid reaction composition include IrCl₃, IrI₃, IrBr₃, [Ir(CO)₂I]₂, [Ir(CO)₂Cl]₂, [Ir(CO)₂Br]₂, [Ir(CO)₂I₂]⁻H⁺, [Ir(CO)₂Br₂]⁻H⁺, [Ir(CO)₂I₄]⁻H⁺, [Ir(CH₃)I₃(CO)₂]⁻H⁺, Ir₄(CO)₁₂, IrCl₃.3H₂O, IrBr₃.3H₂O, Ir₄(CO)₁₂, iridium metal, Ir₂O₃, IrO₂, Ir(acac)(CO)₂, Ir(acac)₃, iridium acetate, [Ir₃O(OAc)₆(H₂O)₃][OAc], and hexachloroiridic acid [H₂IrCl₆], preferably, chloride-free complexes of iridium such as acetates, oxalates and acetoacetates which are soluble in one or more of the carbonylation reaction components such as water, alcohol and/or carboxylic acid. Particularly preferred is green iridium acetate which may be used in an acetic acid or aqueous acetic acid solution.

In the process of the present invention at least one promoter is present in the reaction composition. Suitable promoters are preferably selected from the group consisting of ruthenium, osmium, rhenium, cadmium, mercury, zinc, gallium, indium and tungsten, and are more preferably selected from ruthenium and osmium and most preferably is ruthenium. Preferably, the promoter is present in an effective amount up to the limit of its solubility in the liquid reaction composition and/or any liquid process streams recycled to the carbonylation reactor from the acetic acid recovery stage. The promoter is suitably present in the liquid reaction composition at a molar ratio of promoter : iridium of [0.5 to 15] : 1. As noted above, the beneficial effect of a promoter such as ruthenium has been found to be greatest at the water concentration which gives the maximum carbonylation rate at any defined methyl acetate and methyl iodide concentration. A suitable promoter concentration is 400 to 5000 ppm.

The promoter may comprise any suitable promoter metal-containing compound which is soluble in the liquid reaction composition. The promoter may be added to the liquid reaction composition for the carbonylation reaction in any suitable form which dissolves in the liquid reaction composition or is convertible to soluble form. Examples of suitable ruthenium-containing compounds which may be used as sources of promoter include ruthenium (III) chloride, ruthenium (III) chloride trihydrate, ruthenium (IV) chloride, ruthenium (III) bromide, ruthenium metal, ruthenium oxides, ruthenium (III) formate, [Ru(CO)₃I₃]⁻H⁺, [Ru(CO)₂I₂]ₙ, [Ru(CO)₄I₂], [Ru(CO)₃I₂]₂, tetra(aceto)chlororuthenium(II,III), ruthenium (III) acetate, ruthenium (III) propionate, ruthenium (III) butyrate, ruthenium pentacarbonyl, trirutheniumdodecacarbonyl and mixed ruthenium halocarbonyls such as dichlorotricarbonylruthenium (II) dimer, dibromotricarbonylruthenium (II) dimer, and other organoruthenium complexes such as tetrachlorobis(4-cymene)diruthenium(II), tetrachlorobis(benzene)diruthenium(II), dichloro(cycloocta-1,5-diene)ruthenium (II) polymer and tris(acetylacetonate)ruthenium (III).

Examples of suitable osmium-containing compounds which may be used as sources of promoter include osmium (III) chloride hydrate and anhydrous, osmium metal, osmium tetraoxide, triosmiumdodecacarbonyl, [Os(CO)₄I₂], [Os(CO)₃I₂]₂, [Os(CO)₃I₃]⁻H⁺, pentachloro-µ-nitrodiosmium and mixed osmium halocarbonyls such as tricarbonyldichloroosmium (II) dimer and other organoosmium complexes.

Examples of suitable rhenium-containing compounds which may be used as sources of promoter include Re₂(CO)₁₀, Re(CO)₅Cl, Re(CO)₅Br, Re(CO)₅I, ReCl₃.xH₂O, [Re(CO)₄I]₂, [Re(CO)₄I₂]⁻H⁺ and ReCl₅.yH₂O.

Examples of suitable cadmium-containing compounds which may be used include Cd(OAc)₂, CdI₂, CdBr₂, CdCl₂, Cd(OH)₂, and cadmium acetylacetonate.

Examples of suitable mercury-containing compounds which may be used as sources of promoter include Hg(OAc)₂, HgI₂, HgBr₂, HgCl₂, Hg₂I₂, and Hg₂Cl₂.

Examples of suitable zinc-containing compounds which may be used as sources of promoter include Zn(OAc)₂, Zn(OH)₂, ZnI₂, ZnBr₂, ZnCl₂, and zinc acetylacetonate.

Examples of suitable gallium-containing compounds which may be used as sources of promoter include gallium acetylacetonate, gallium acetate, GaCl₃, GaBr₃, GaI₃, Ga₂Cl₄ and Ga(OH)₃.

Examples of suitable indium-containing compounds which may be used as sources of promoter include indium acetylacetonate, indium acetate, InCl₃, InBr₃, InI₃, InI and In(OH)₃.

Examples of suitable tungsten-containing compounds which may be used as sources of promoter include W(CO)₆, WCl₄, WCl₆, WBr₅, WI₂, or C₉H₁₂ W(CO)₃ and any tungsten chloro-,bromo- or iodo-carbonyl compound.

Preferably, the iridium- and promoter-containing compounds are free of impurities which provide or generate in situ ionic iodides which may inhibit the reaction, for example, alkali or alkaline earth metal or other metal salts.

Ionic contaminants such as, for example, (a) corrosion metals, particularly nickel, iron and chromium and (b) phosphines or nitrogen containing compounds or ligands which may quaternise in situ; should be kept to a minimum in the liquid reaction composition as these will have an adverse effect on the reaction by generating I⁻ in the liquid reaction composition which has an adverse effect on the reaction rate. Some corrosion metal contaminants such as for example molybdenum have been found to be less susceptible to the generation of I⁻. Corrosion metals which have an adverse affect on the reaction rate may be minimised by using suitable corrosion resistant materials of construction. Similarly, contaminants such as alkali metal iodides, for example lithium iodide, should be kept to a minimum. Corrosion metal and other ionic impurities may be reduced by the use of a suitable ion exchange resin bed to treat the reaction composition, or preferably a catalyst recycle stream. Such a process is described in US 4007130. Preferably, ionic contaminants are kept below a concentration at which they would generate 500 ppm I⁻, preferably less than 250 ppm I⁻ in the liquid reaction composition.

The carbon monoxide reactant may be essentially pure or may contain inert impurities such as carbon dioxide, methane, nitrogen, noble gases, water and C₁ to C₄ paraffinic hydrocarbons. The presence of hydrogen in the carbon monoxide feed and generated in situ by the water gas shift reaction is preferably kept low as its presence may result in the formation of hydrogenation products. Thus, the amount of hydrogen in the carbon monoxide reactant is preferably less than 1 mol %, more preferably less than 0.5 mol % and yet more preferably less than 0.3 mol % and/or the partial pressure of hydrogen in the carbonylation reactor is preferably less than 1 bar partial pressure, more preferably less than 0.05 MPa (0.5 bar) and yet more preferably less than 0.03 MPa (0.3 bar). The partial pressure of carbon monoxide in the reactor is suitably in the range 0.1 to 70 MPa (1 to 70 bar), preferably 0.1 to 3.5 MPa (1 to 35 bar), more preferably 0.1 to 15 MPa (1 to 15 bar).

The total pressure of the carbonylation reaction is suitably in the range 1.0 to 20.0 MPag (10 to 200 barg), preferably 1.5 to 100 MPag (15 to 100 barg), more preferably 1.5 to 5.0 MPag (15 to 50 barg). The temperature of the carbonylation reaction is suitably in the range 100 to 300 °C, preferably in the range 150 to 220 °C.

The process of the present invention is preferably performed as a continuous process.

The acetic acid product may be recovered from the liquid reaction composition by withdrawing vapour and/or liquid from the carbonylation reactor and recovering acetic acid from the withdrawn material. Preferably, acetic acid is recovered from the liquid reaction composition by continuously withdrawing liquid reaction composition from the carbonylation reactor and recovering acetic acid from the withdrawn liquid reaction composition by one or more flash and/or fractional distillation stages in which the acetic acid is separated from the other components of the liquid reaction composition such as iridium catalyst, methyl iodide co-catalyst, promoter, methyl acetate, unreacted methanol, water and acetic acid solvent which may be recycled to the reactor to maintain their concentrations in the liquid reaction composition. To maintain stability of the iridium catalyst during the acetic acid product recovery stage, water in process streams containing iridium carbonylation catalyst for recycle to the carbonylation reactor should be maintained at a concentration of at least 0.5 % by weight.

A particularly preferred liquid reaction composition comprises about 5 % by weight water, about 7 % by weight methyl iodide co-catalyst, about 15 % by weight methyl acetate, iridium catalyst at a concentration in the range 400 to 3000 ppm measured as iridium to give a rate of carbonylation reaction in the range of 10 to 40 mol/l/hr at a carbonylation reaction temperature of about 189 °C and a carbonylation reaction pressure of 2.2 to 3.0 MPag (22 to 30 barg) and carbon monoxide partial pressure of 0.4 to 1.2 MPa (4 to 12 bar), ruthenium promoter at a concentration in the range 400 to 4000 ppm measured as ruthenium to give a molar ratio of ruthenium : iridium of approximately 2.5 : 1 and the balance of the reaction composition comprising substantially acetic acid. Higher or lower concentrations of catalyst and/or higher or lower temperatures and/or higher or lower partial pressures of carbon monoxide may be used to obtain higher or lower rates of reaction.

The invention will now be illustrated by way of example only and with reference to the following examples and drawings in which Figures 1 to 6 represent the effect of water concentration on carbonylation rate in batch autoclave experiments. Figure 7 shows in schematic form apparatus used to illustrate the process of the present invention in continuous operation. Figure 8 represents the effect of water concentration on carbonylation rate in a continuous reactor.

### Batch Carbonylation Experiments

The following batch experiments were performed to illustrate the present invention. Reaction components were charged to an autoclave with an amount of carbonylatable reactant (methyl acetate) which was consumed during the course of the reaction. In the batch carbonylation experiments the concentration of water decreased as the carbonylation progressed; the carbonylation of methyl acetate and consumption of water being equivalent to the carbonylation of methanol.

By monitoring the rate of carbonylation reaction and calculating the concentration of the reaction components during the experiment, it is possible to determine the rate of carbonylation reaction which would be expected if a carbonylation process were to be operated continuously whilst maintaining under steady state, a liquid reaction composition which is the same as the total reaction composition calculated at any particular point in the batch experiment. In the batch experiments the term 'reaction composition' refers to the total composition of the components in the autoclave in the cold degassed state. In the continuous experiments below, the liquid reaction composition was analysed. The principal difference between the batch experiments and continuous operation is that in the batch experiments, no allowance was made in calculating the component concentrations, for partitioning of the reaction components between the liquid and gaseous phases. Owing to this partitioning, the concentration of the reaction components present in the liquid phase in a batch reaction under reaction conditions was similar, but not identical, to the total reaction composition. In particular, the more volatile components in the reaction composition, such as methyl iodide and methyl acetate, were slightly less concentrated in the liquid reaction composition than in the total reaction composition, whereas the water concentration was comparable between the two. Therefore, the rate calculated in a batch experiment at a certain total reaction composition will be similar to that in a continuous process operating with a liquid composition which is the same as the batch total reaction composition. In addition, trends observed in batch experiments by varying the process variables, such as water concentration, were comparable with the trends observed in continuous experiments. All batch carbonylation experiments were performed using a 300 ml zirconium autoclave reactor equipped with a Dispersimax (Trade Mark) stirrer, liquid catalyst injection facility and cooling coils. A gas supply to the autoclave was provided from a ballast vessel, feed gas being provided to maintain the autoclave at a constant pressure. The rate of gas uptake at a certain point in a reaction run was used to calculate the carbonylation rate, as number of moles of reactant consumed per litre of cold degassed reactor composition per hour (mol/l/hr), at a particular reactor composition (total reactor composition based on a cold degassed volume).

The methyl acetate concentration was calculated during the course of the reaction from the starting composition, assuming that one mole of methyl acetate was consumed for every mole of carbon monoxide that was consumed. No allowance was made for organic components in the autoclave headspace.

For each batch carbonylation experiment the catalyst, H₂IrCl₆, dissolved in a portion of the acetic acid / water liquid autoclave charge, was charged to the liquid injection facility. If a promoter was used, it was placed in the autoclave with a portion (10g) of the acetic acid charge. The autoclave was then pressure tested with nitrogen, vented via a gas sampling system, and flushed with carbon monoxide several times (3 times at 0.3-1.0 MPag) [(3 times at 3-10 barg)]. The remaining liquid components of the reaction composition were charged to the autoclave via a liquid addition port. The autoclave was then pressurised with carbon monoxide (typically 0.6 MPag) [(typically 6 barg)] and heated with stirring (1500 rpm) to reaction temperature, 190°C. The total pressure was then raised to approximately 0.3 MPag (3 barg) below the desired operating pressure by feeding carbon monoxide from the ballast vessel. Once stable at temperature (about 15 minutes) the catalyst was injected using an over pressure of carbon monoxide. The iridium concentrations quoted in these batch experiments were based upon a catalyst inject efficiency of 92%. The reactor pressure was maintained at a constant value (±0.05 MPag) [(± 0.5 barg)] by feeding gas from the ballast vessel throughout the experiment. Gas uptake from the ballast vessel was measured using datalogging facilities throughout the course of the experiment. The reaction temperature was maintained within ± 1°C of the desired reaction temperature by means of a heating mantle connected to a Eurotherm (Trade Mark) control system. In addition, excess heat of reaction was removed by means of cooling coils. Each experiment was conducted until the gas uptake had ceased. The ballast vessel was then isolated and the reactor crash cooled by use of the cooling coils.

H₂IrCl₆ (22.2 % w/w Ir aqueous solution) was supplied by Johnson Matthey. The acetic acid was obtained from carbonylation of a mixed methanol/methyl acetate feedstock and contained very low amounts of propionic acid and its precursors. Methyl acetate (29, 699-6), water (32, 007-2) and methyl iodide (I-850-7) were supplied by Aldrich. [Ru(CO)₄I₂] was synthesised from Ru₃(CO)₁₂ (STREM) and iodine (Aldrich, 37,655-8) and was stored under a carbon monoxide atmosphere in a freezer in a Schlenk tube prior to use.

Examples 1 to 12 demonstrate the effect of water concentration, expressed in % by weight, on carbonylation reaction rate using a ruthenium promoted iridium catalyst (molar ratio of ruthenium : iridium approximately 2 :1) at 190°C and 2.8 MPag (28 barg) total pressure. Charge compositions are given in Table 1. Rate data, at calculated methyl acetate concentrations in the total reaction composition (as expressed above, cold degassed liquid) of 30%, 25%, 20% ,15% ,10%, 7.5% and 5% by weight, are given in Table 2. The carbonylation rate has been calculated at various methyl acetate and water concentrations and the data according to the present invention (water concentration from 1 to 6.5 % by weight of total reaction composition, cold degassed liquid) are separated from comparative data by a thick solid black line in the Table. From the data it is expected that a continuous carbonylation process could be operated at steady state conditions with a liquid reaction composition the same as the total reaction composition calculated for the batch autoclave experiments with a similar rate of carbonylation achieved.

**Table 1**

| **Charge compositions for ruthenium promoted reactions in a 300 ml zirconium batch autoclave.** | | | | | | | |
|---|---|---|---|---|---|---|---|
| Example | Ref. | Methyl acetate (g) | Acetic acid (g) | Methyl iodide (g) | Water (g) | H₂IrCl₆ (g)^{a} | Ru(CO)₄I₂ (g) |
| 1 | 637 | 60.02 | 45.65 | 13.96 | 28.30 | 0.641 | 1.465 |
| 2 | 636 | 60.00 | 53.84 | 13.97 | 20.16 | 0.643 | 1.473 |
| 3 | 652 | 60.16 | 58.08 | 13.96 | 15.97 | 0.641 | 1.460 |
| 4 | 656 | 60.03 | 60.43 | 13.96 | 13.51 | 0.643 | 1.463 |
| 5 | 633 | 60.03 | 62.00 | 13.96 | 11.95 | 0.640 | 1.465 |
| 6 | 654 | 60.02 | 62.11 | 13.96 | 11.95 | 0.643 | 1.460 |
| 7 | 655 | 60.04 | 62.16 | 13.97 | 11.96 | 0.643 | 1.466 |
| 8 | 635 | 60.01 | 62.04 | 13.96 | 11.74 | 0.641 | 1.464 |
| 9 | 650 | 60.14 | 64.38 | 13.97 | 9.50 | 0.645 | 1.466 |
| 10 | 658 | 60.07 | 66.01 | 13.95 | 7.96 | 0.641 | 1.462 |
| 11 | 651 | 60.07 | 67.61 | 13.94 | 6.41 | 0.642 | 1.460 |
| 12 | 659 | 60.03 | 69.16 | 13.96 | 4.85 | 0.643 | 1.459 |
| 13 | 648 | 60.17 | 55.51 | 20.50 | 11.91 | 0.642 | 1.467 |
| 14 | 660 | 60.04 | 43.54 | 13.95 | 28.31 | 0.641 | 3.650 |
| 15 | 661 | 60.06 | 55.75 | 13.96 | 16.03 | 0.645 | 3.650 |
| 16 | 632 | 59.98 | 59.85 | 13.96 | 11.97 | 0.639 | 3.651 |

| | | | | | | | |
|---|---|---|---|---|---|---|---|
| (a) Weight expressed as pure H₂IrCl₆. | | | | | | | |

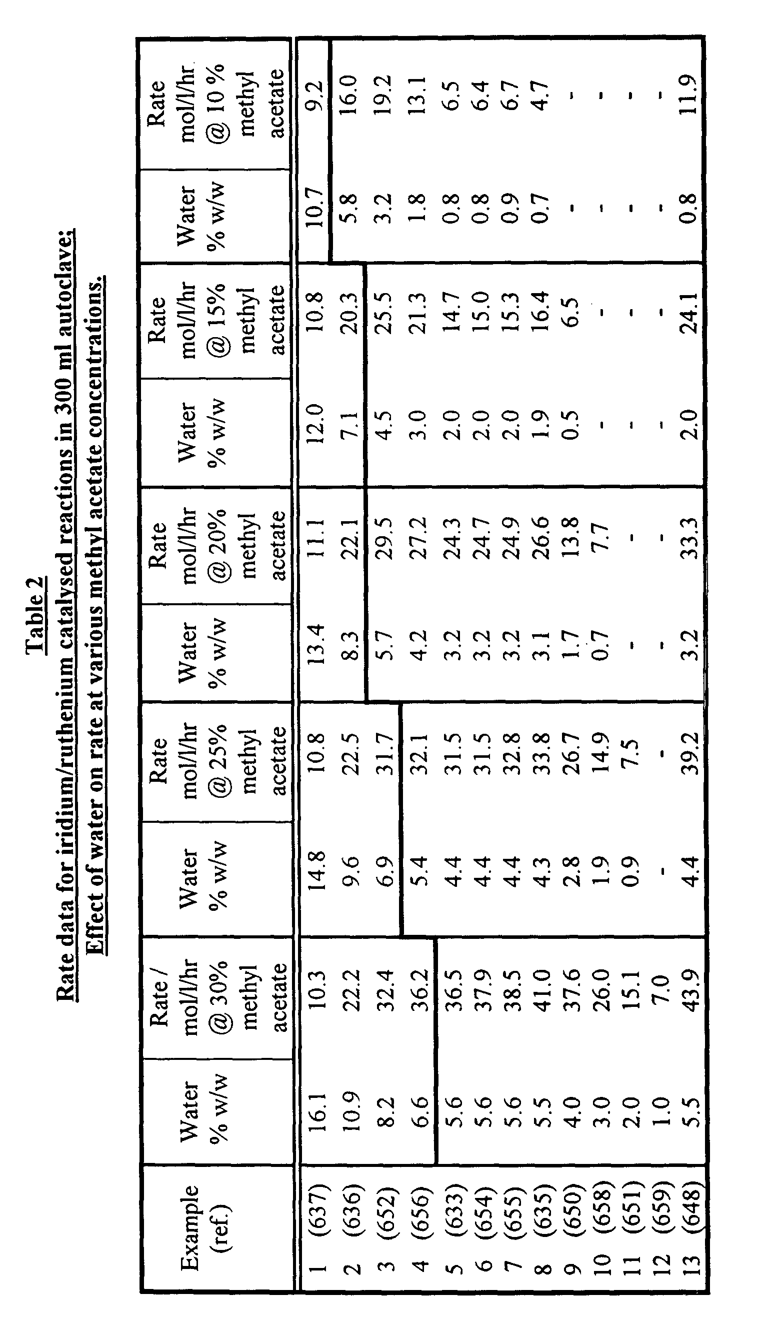

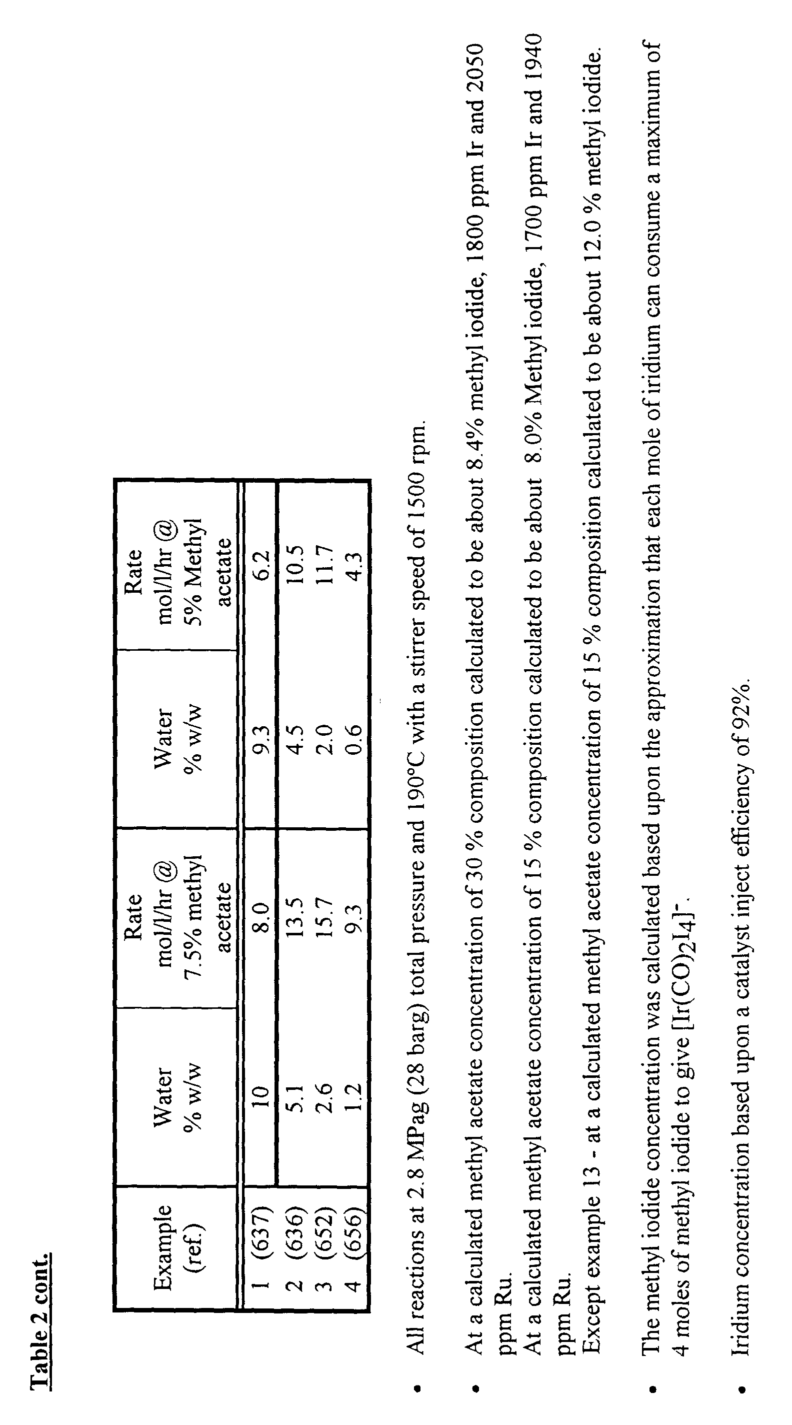

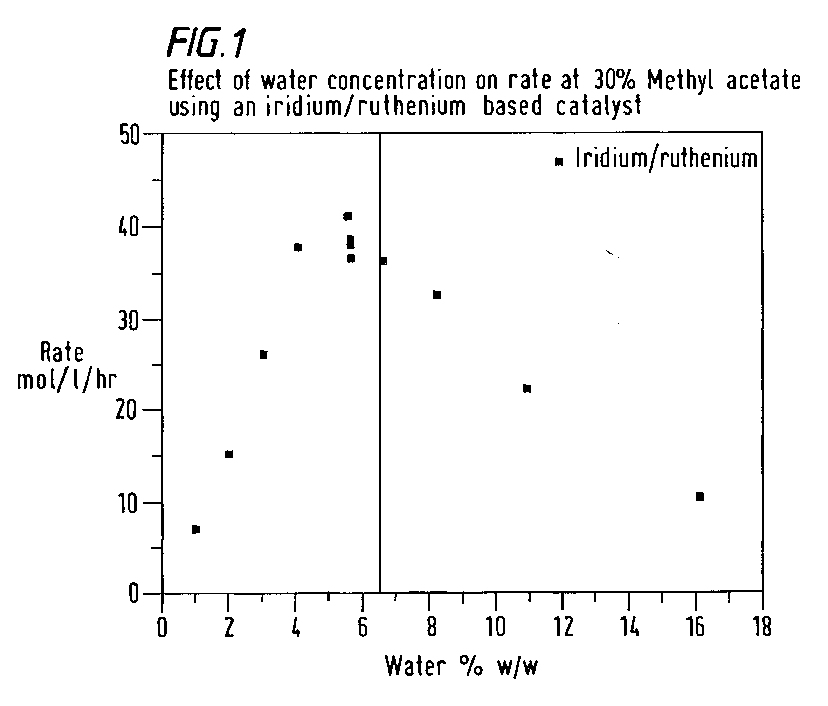

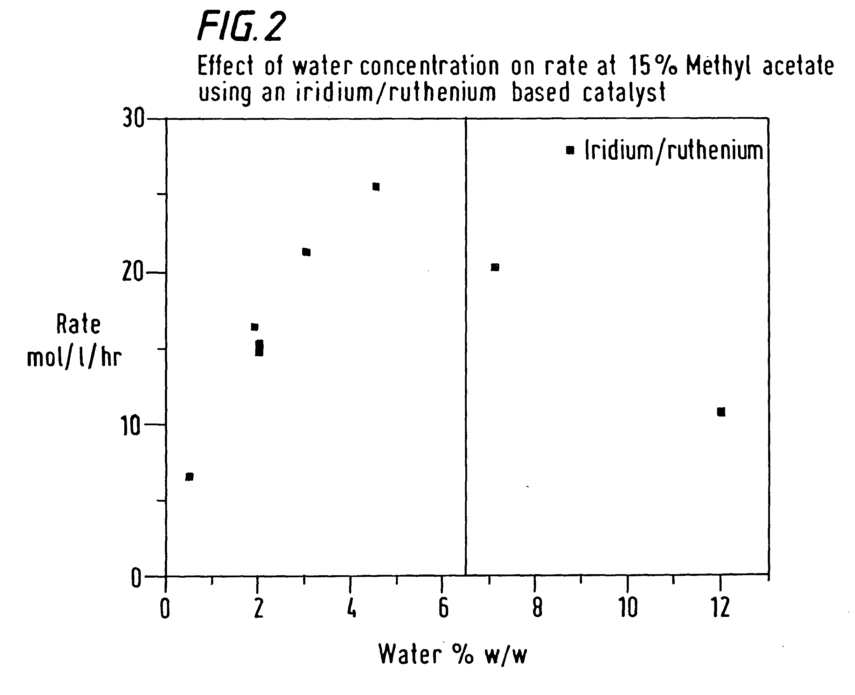

Figures 1 and 2 show some of the data from Table 2 in graph form and illustrate the effect of water concentration on rate, at 30% and 15% w/w methyl acetate respectively, for iridium/ruthenium catalysed methanol carbonylation.

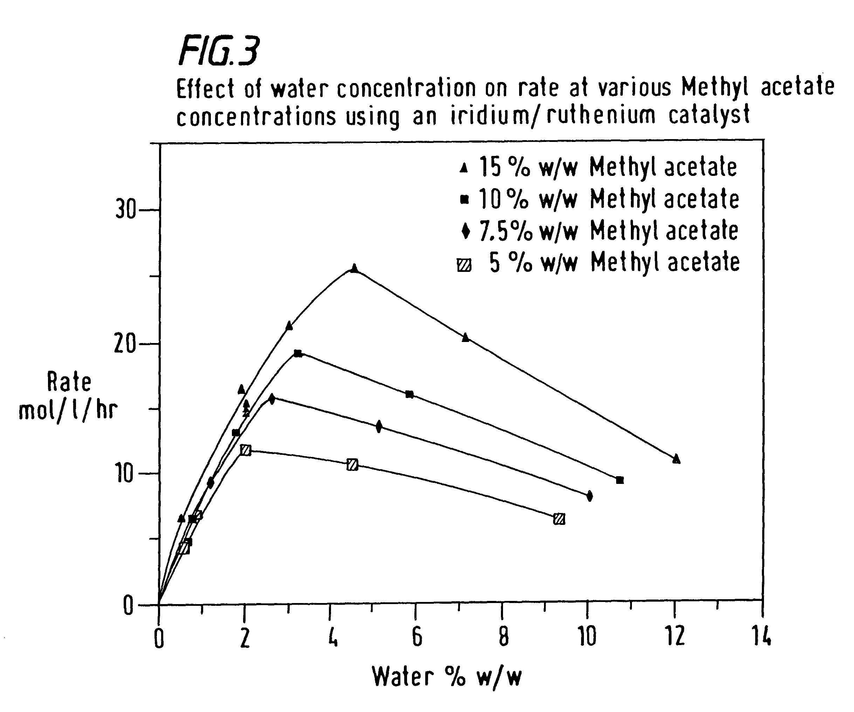

Figure 3 shows the data from Table 2 at various methyl acetate concentrations of 5, 7.5, 10 and 15 % by weight.

Further experiments were performed without ruthenium promoter. Experiments A to J demonstrate the effect of water concentration, (expressed as % by weight of total reaction composition, cold degassed liquid), on carbonylation reaction rate using an iridium only catalyst without ruthenium promoter at 190°C and 2.8 MPag (28 barg) total pressure. Charge compositions are given in Table 3. Rate data, at 30%, 25%, 20% ,15%, 10%, 7.5% and 5% by weight calculated methyl acetate concentration, (expressed as % by weight of total reaction composition, cold degassed liquid) are given in Table 4.

**Table 3**

| **Charge compositions for reactions in a 300 ml zirconium batch autoclave.** | | | | | | |
|---|---|---|---|---|---|---|
| Experiment | Ref. | Methyl acetate (g) | Acetic acid (g) | Methyl iodide (g) | Water (g) | H₂IrCl₆ (g)^{a} |
| A | 630 | 60.07 | 47.13 | 13.96 | 28.30 | 0.639 |
| B | 609 | 59.99 | 55.32 | 13.97 | 20.11 | 0.640 |
| C | 641 | 60.01 | 59.40 | 13.96 | 16.06 | 0.641 |
| D | 653 | 60.02 | 59.52 | 13.97 | 16.00 | 0.643 |
| E | 598 | 59.99 | 63.54 | 13.97 | 11.94 | 0.641 |
| F | 615 | 60.02 | 63.51 | 13.96 | 11.96 | 0.640 |
| G | 621 | 59.99 | 63.49 | 13.96 | 11.96 | 0.640 |
| H | 634 | 60.05 | 63.49 | 13.96 | 11.98 | 0.649 |
| I | 640 | 60.03 | 65.95 | 13.97 | 9.51 | 0.644 |
| J | 643 | 60.01 | 66.15 | 13.96 | 9.52 | 0.646 |
| K | 642 | 60.02 | 68.99 | 13.96 | 6.46 | 0.642 |
| L | 604 | 60.00 | 57.04 | 20.56 | 11.96 | 0.641 |

| | | | | | | |
|---|---|---|---|---|---|---|
| (a) Weight expressed as pure H₂IrCl₆. | | | | | | |

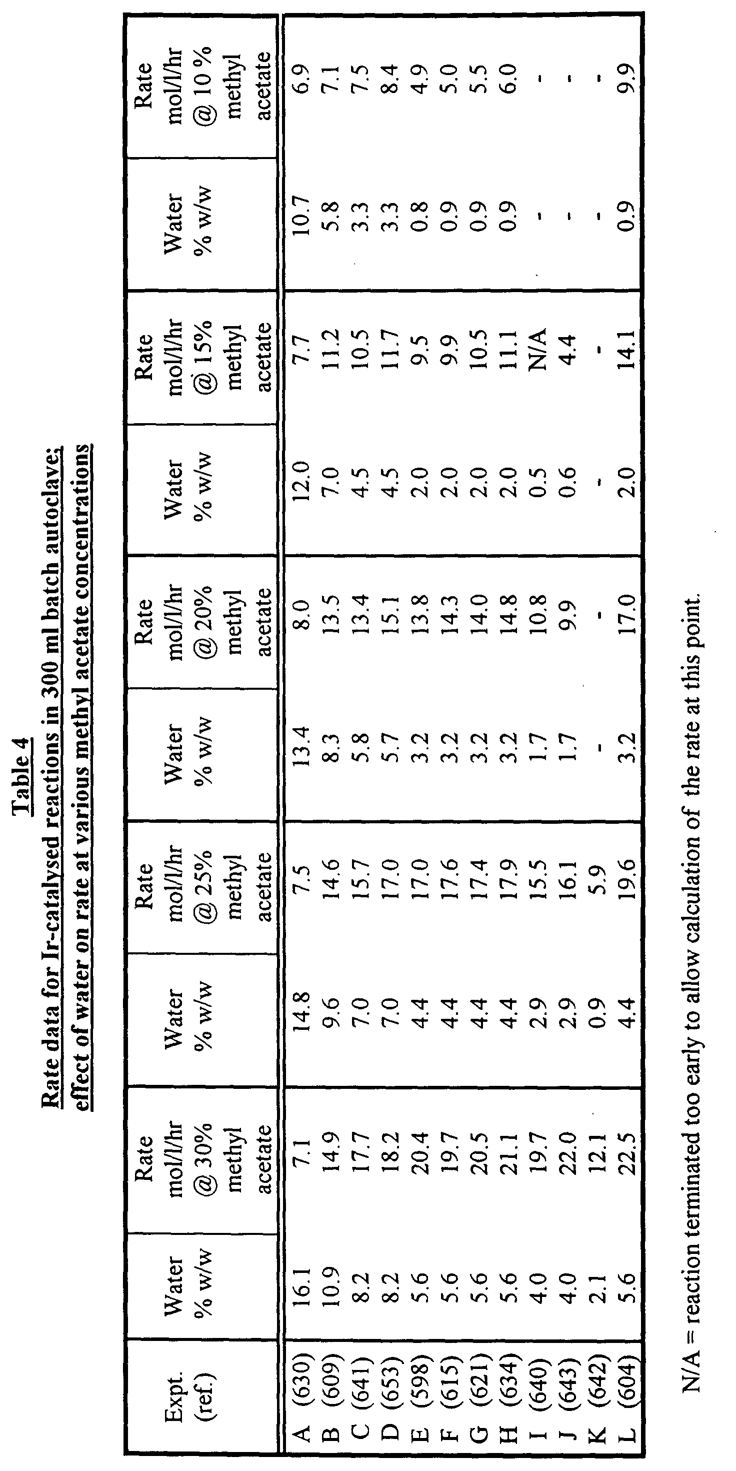

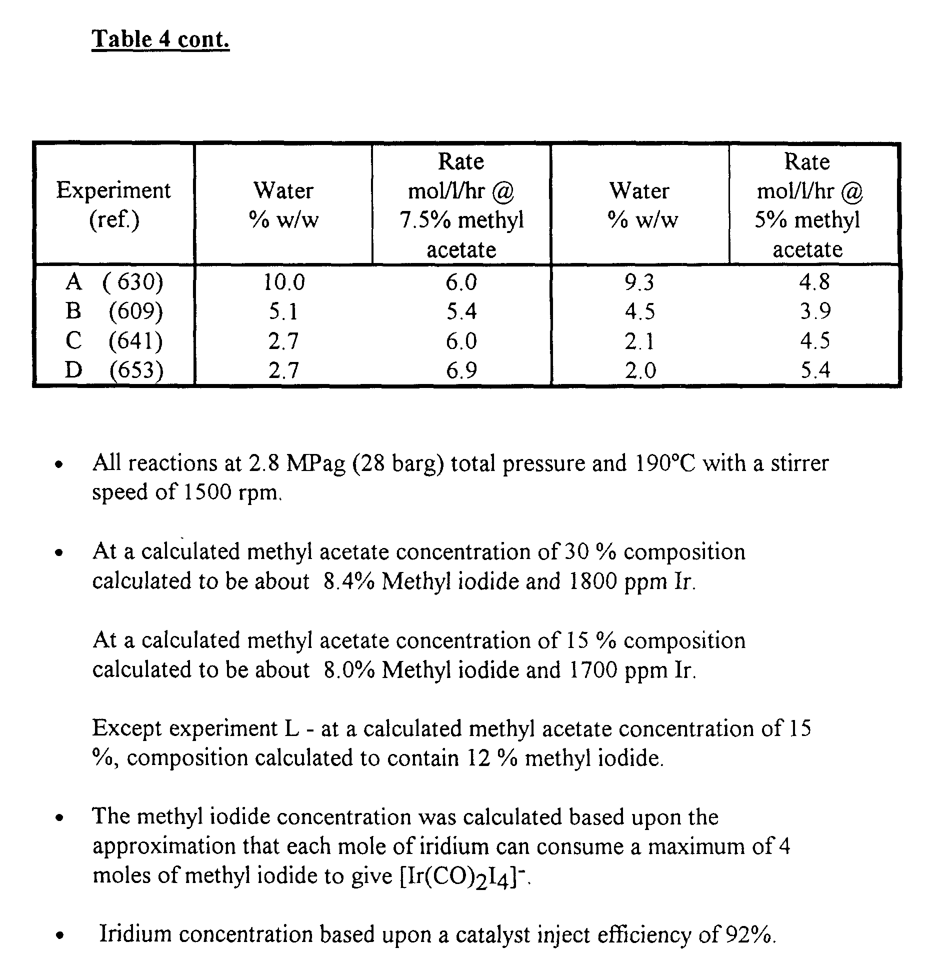

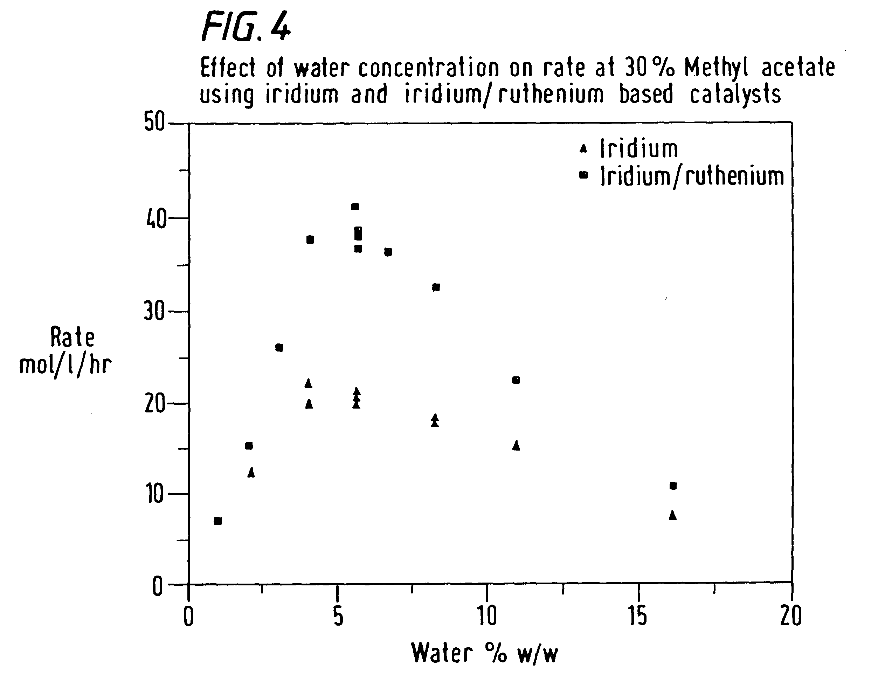

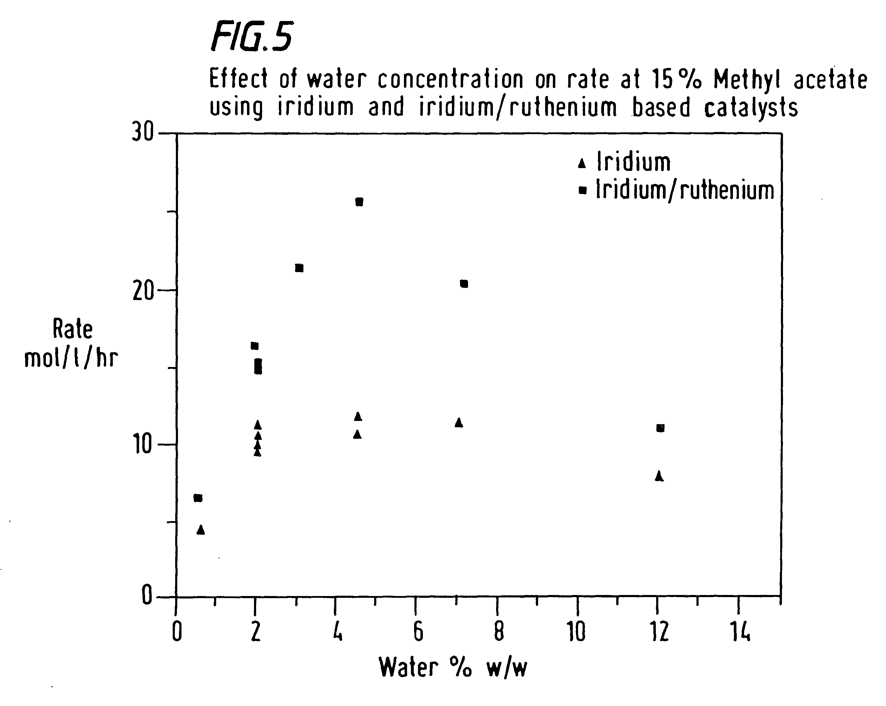

Comparison of the data for Table 4 for iridium only with that for Table 2 for iridium/ruthenium is shown in graph form in Figures 4 and 5.

A comparison of the promotional effect of ruthenium, at 30% w/w methyl acetate, at various water concentrations is given in Table 5 below (Experiments A, B, D, H, J & K are compared with Examples 1, 2, 3, 7, 9 & 11).

**Table 5**

| Water concentration % w/w | Carbonylation rate mol/l/hr | | Ruthenium rate promotion % |
|---|---|---|---|
| | Ir catalyst only | Ir catalyst and Ru promoter | |
| 16.1 | 7.1 | 10.3 | 45% |
| 10.9 | 14.9 | 22.2 | 49% |
| 8.2 | 18.2 | 32.4 | 78% |
| 5.6 | 21.1 | 38.5 | 83% |
| 4.0 | 22.0 | 37.6 | 71% |
| 2.1&2.0 | 12.1 | 15.1 | 25% |

It can be seen from Tables 2, 4 and 5 and Figures 1 to 5 that as the water concentration was reduced from greater than 6.5 % by weight, the carbonylation rate increased, passing through a maximum, and then declined as very low water levels were approached, for both the iridium and iridium/ruthenium catalyst systems. It is also apparent that ruthenium became more effective as a promoter as the water concentration was reduced, ruthenium being most effective in promotional terms when maximum rates were also observed which was at a water concentration no greater than 6.5 % by weight. At lower water levels, at 30% methyl acetate, the rate declined as did the promotional effect of ruthenium.

Figure 5 and Table 6 below (comparison of Experiments A, B, D, & H with Examples 1, 2, 3, & 7) illustrate the same point but at a lower methyl acetate concentration of 15% by weight.

**Table 6**

| Water concentration % w/w | Carbonylation rate mol/l/hr | | Ruthenium rate promotion % |
|---|---|---|---|
| | Ir catalyst only | Ir catalyst and Ru promoter | |
| 12.0 | 7.7 | 10.8 | 40% |
| 7.0 & 7.1 | 11.2 | 20.3 | 81% |
| 4.5 | 11.7 | 25.5 | 118% |
| 2.0 | 11.1 | 15.3 | 38% |

Figure 3 illustrates that as the methyl acetate concentration is reduced in the process of the present invention from 15% through to 5% by weight calculated methyl acetate concentration in the total reaction composition, the optimum water concentration, in terms of rate, moves to a lower concentration.

It is also apparent from Tables 2 & 4 that at 5% by weight calculated methyl acetate concentration in the total reaction composition, and a relatively low water concentration of 2% by weight ( see Example 3) a relatively high carbonylation rate of 11.7 mol/l/hr was observed as compared with the unpromoted Experiment D, which gave a rate of 5.4 mol/l/hr i.e. ruthenium promotion under these conditions was large (117% rate promotion) even at 2% by weight water. Similarly, at 3.2% by weight water and 10% weight methyl acetate a relatively high rate of 19.2 mol/l/hr was observed (Example 3).

Further batch experiments 13 to 16 and L were performed. Charge compositions are shown in Tables 1 and 3. Results for experiment L are shown in Table 4, for Example 13 in Table 2 and for Examples 14 to 16 in Table 7.

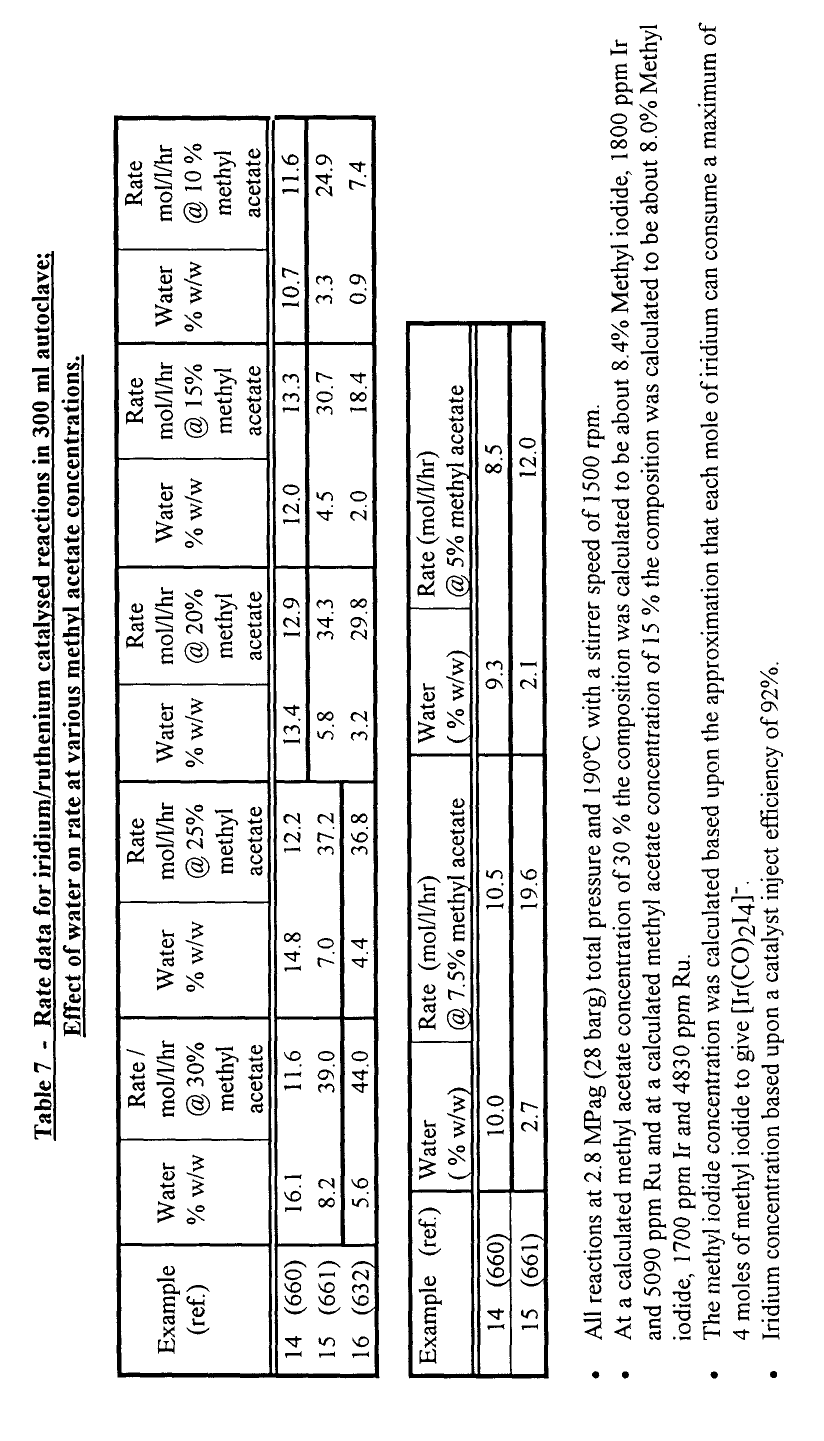

Experiment L shows the effect of increasing methyl iodide co-catalyst concentration for an iridium-only catalysed reaction without promoter. By comparison with experiment H in Table 4, it can be seen that increasing methyl iodide has a beneficial effect on rate of reaction, especially at low water concentration.

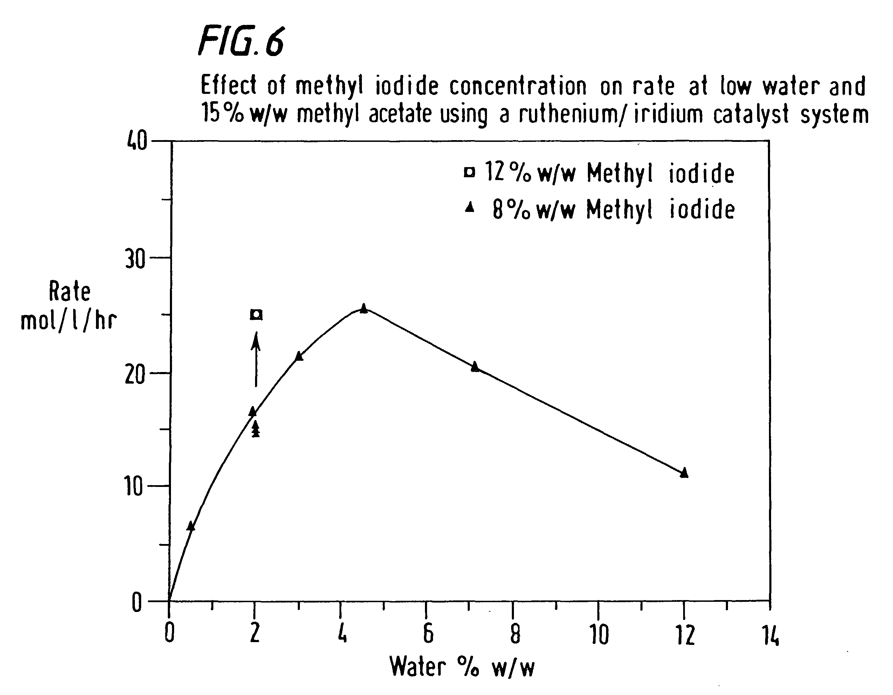

Example 13 (Table 2) shows the effect of increasing methyl iodide co-catalyst concentration from about 8 % by weight to 12 % by weight for a ruthenium-promoted, iridium-catalysed process by comparison with Example 7. It is also apparent by comparison with Experiment L that, at low water, the promotional effect of ruthenium was increased when the methyl iodide concentration was raised from 8 % to 12 % (compare experiment H with Example 7), as show in Table 8 below. The effect of increasing methyl iodide concentration from 8 to 12 % by weight at 15 % methyl acetate is also shown in graph form in Figure 6.

**Table 8**

| Experiment | Methyl iodide concentration (% by weight) | Catalyst | Reaction rate (mol/l/hr) | Ruthenium promotion (%) |
|---|---|---|---|---|
| H | 8 | iridium | 11.1 | 38 |
| 7 | 8 | iridium & ruthenium | 15.3 | |
| L | 12 | iridium | 14.1 | 71 |
| 13 | 12 | iridium & ruthenium | 24.1 | |

Comparison of experiment H with experiment L (27 % increase in reaction rate in going from 8 to 12 % methyl iodide) and of Example 7 with Example 13 (58% increase in reaction rate in going from 8 to 12 % methyl iodide), shows that under these conditions, the ruthenium promoted iridium catalyst was more sensitive towards methyl iodide concentration than the unpromoted catalyst.

Examples 14 to 16 illustrate the effect of water concentration at various methyl acetate concentrations using ruthenium : iridium molar ratio of about 5 : 1, at 8 % methyl iodide. These examples also demonstrate the additional benefit on reaction rate of increasing the ruthenium : iridium molar ratio from about 2 :1 (Examples 1 to 12) to 5 : 1

### Continuous Carbonylation Reactor Examples

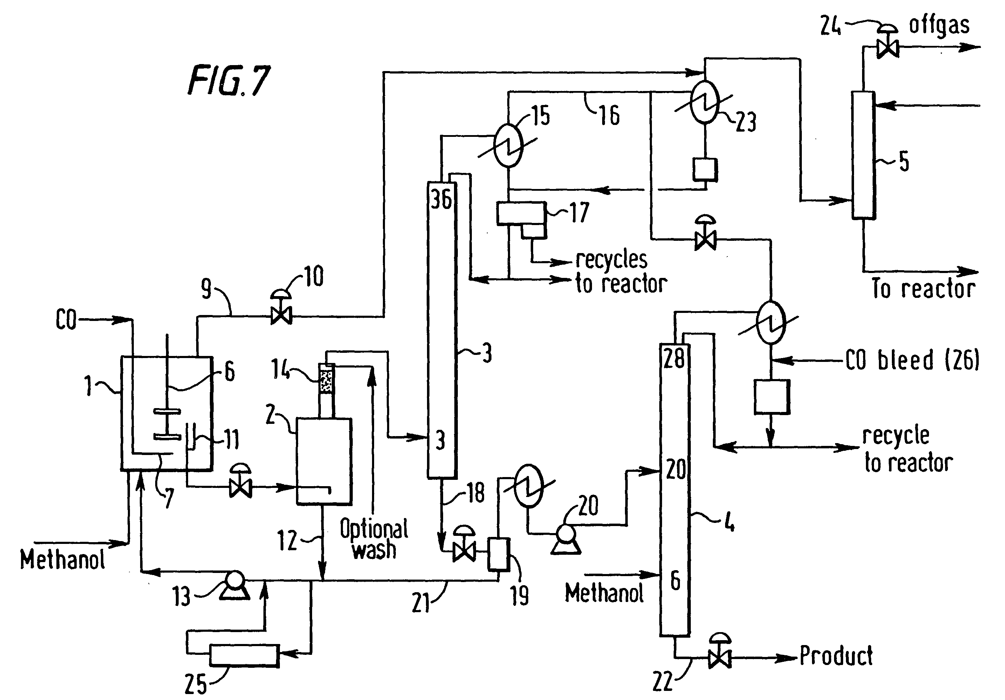

Experiments were performed to illustrate the present invention using a carbonylation reactor operating continuously at a steady state liquid reaction composition. A schematic diagram of the apparatus is given in Figure 7.
The apparatus comprised a stirred carbonylation reactor (1), a flash tank (2) and two distillation columns (3,4) all constructed from zirconium 702. It also had two packed off-gas scrubbers: an optional acetic acid scrubber (not shown) and a methanol scrubber (5) constructed from stainless steel.

In use, commercial grade methanol, which has been used to scrub the off-gas was carbonylated in the 6 litre reactor (1) in the presence of the iridium carbonylation catalyst and promoter at a pressure of 2.40-3.00 MPag (24.0 - 30.0 barg) and a temperature of 181-195°C. The reactor(1) was fitted with a stirrer/propeller (6) and a baffle cage (not shown) to ensure intimate mixing of the liquid and gaseous reactants. Carbon monoxide was supplied from a commercial plant or from pressure bottles to the reactor via a sparge (7) fitted beneath the stirrer (6). To minimise iron ingress into the reactor the carbon monoxide passed through a carbon filter (not shown). A jacket (not shown), through which hot oil was circulated, enabled the reaction liquid in the reactor to be maintained at a constant reaction temperature. The liquid reaction composition was analysed by near infra red analysis or by gas chromatography.

To purge inerts, high pressure off-gas was removed from the reactor at a constant flow rate through line (9). It passed through a condenser (not shown) before the pressure was dropped to 0.148 MPag (1.48 barg) across valve (10) for it to be fed into the scrubbing system.

Liquid reaction composition was withdrawn from the carbonylation reactor down a still well (11) and fed into the flash tank (2) under reactor level control. In the flash tank the liquid reaction composition was flashed down to a pressure of 0.148 MPag (1.48 barg). The resulting mixture of vapour and liquid was separated: the catalyst- rich liquid returning to the reactor by line (12) and pump (13) and the vapour was passed through a demister (14) and then directly into the light ends distillation column (3), as a vapour. The demister consisted of two parts. The first was a gauze demister section, the second was a packed section. This second section was optionally washed with aqueous material from the head of the light ends column. An ion exchange corrosion metal removal bed (25) was operated to remove corrosion metals from a slip stream of the recycled liquid fraction from the flash tank (catalyst recycle stream) and thereby maintain the concentration of corrosion metals below 100 ppm in the liquid reaction composition.

The acetic acid product recovery section of the apparatus comprised a 36-plate light ends distillation column (3), and a 28-plate drying distillation column (4). Both were fabricated from zirconium with PTFE sieve trays.

Distillation column (3) was operated at the same pressure as the flashtank (about 0.148 MPag [1.48 barg]) with a vapour feed. It had 3 trays below the feed point.

Distillation column (4) operated at 1.8 barg head pressure with a liquid feed at tray 20 counted from the base. The pressure was maintained by using a bleed (26) of carbon monoxide into the head of the column. To minimise heat losses from the columns they were lagged, trace heated and lagged. The trace heating was controlled to a temperature similar to the process temperature at that point within the column.

The overheads from distillation column (3) were passed through a condenser (15); the low pressure off gas passing along line (16) to the scrubbing system, and the condensed liquid falling into a decanter (17). This liquid was two-phase; the heavier organic layer was pumped directly back to the reactor and the lighter aqueous layer was split, some was returned to the top of the column as reflux and some optionally used as the wash to the flashtank and thence to the reactor. The remainder of the aqueous phase was returned to the reactor via. the catalyst recycle pump (13). The distillation column (3) had an electrically-heated, thermo-siphon reboiler (not shown), with boil-up controlled on temperature in the reboiler liquid. Crude acetic acid was removed from the reboiler along line 18 and flashed into a vaporiser (19).

The vaporiser was constructed from zirconium 702 and operated at atmospheric pressure with the boil up controlled on the level in the vaporiser. The majority of the material introduced into the vaporiser was removed from the vaporiser overhead as a vapour and was condensed before being pumped by pump (20) into the drying column (4). The feed to the drying column may also be as a vapour. A base bleed from the vaporiser was recycled along line (21) to the reaction system.

It was also possible to operate the distillation column (3) with a vapour take-off from the base with a kettle bleed in place of the vapouriser.

Drying column (4) was heated and controlled in a similar way to column (3), but with boil-up controlled by the temperature on tray 8. Its overhead material was single phase. Some material from the overhead was used as reflux for the distillation column and the remainder was returned to the reactor. Dry acetic acid product was removed from the base of the column along line (22). The concentration of propionic acid in the dry acetic acid product from the base of the drying column was indicative of the rate of production of propionic acid by-product. Methanol was fed into the column at tray 6, to react with hydroiodic acid.

The low pressure off gas from columns (3, 4) was first passed through the low pressure off gas fridge condensers (23), condensed liquid being returned to the decanter (17). The resultant vapour was joined with the pressure-reduced high pressure off gas before entering the base of the methanol scrubber (5).

The methanol off gas scrubber (5), contained "knitmesh" packing. It used chilled methanol to remove the methyl iodide from the off gas stream. The methanol scrubbate from the scrubber was combined with fresh methanol to provide the reactor feed. The scrubbed off-gas passed through a control valve (24), which controlled the pressure in the flashtank, and was analysed before passing via. a vent to atmosphere.

The off-gas: high pressure, low pressure or combined could be passed through an acetic acid scrubber (not shown), prior to the methanol scrubber. This scrubber was similar to the methanol scrubber but it contained Hastelloy B2 "knitmesh" packing and used ca. 10% of the acetic acid product to scrub the off-gas. The scrubbate is then returned to the flashtank. If the acetic acid scrubber was used for the low pressure off gas, the fridge condensers were by-passed.

The results for operation with various liquid reaction compositions are shown in Table 9.

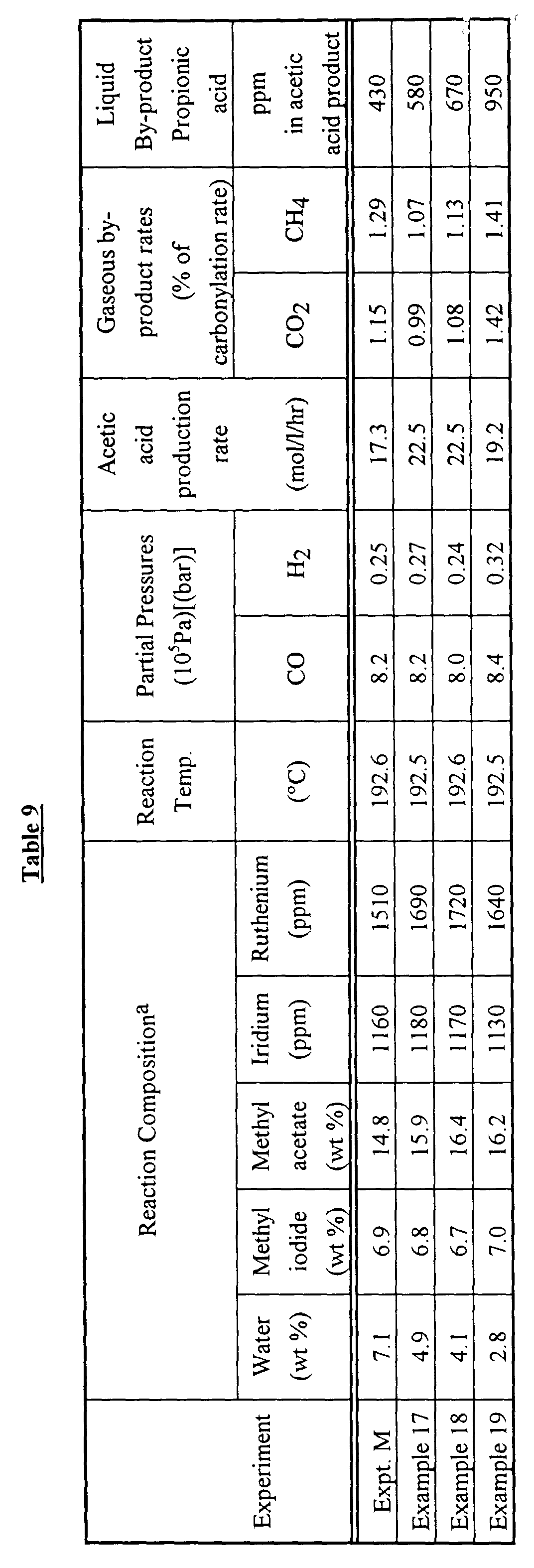

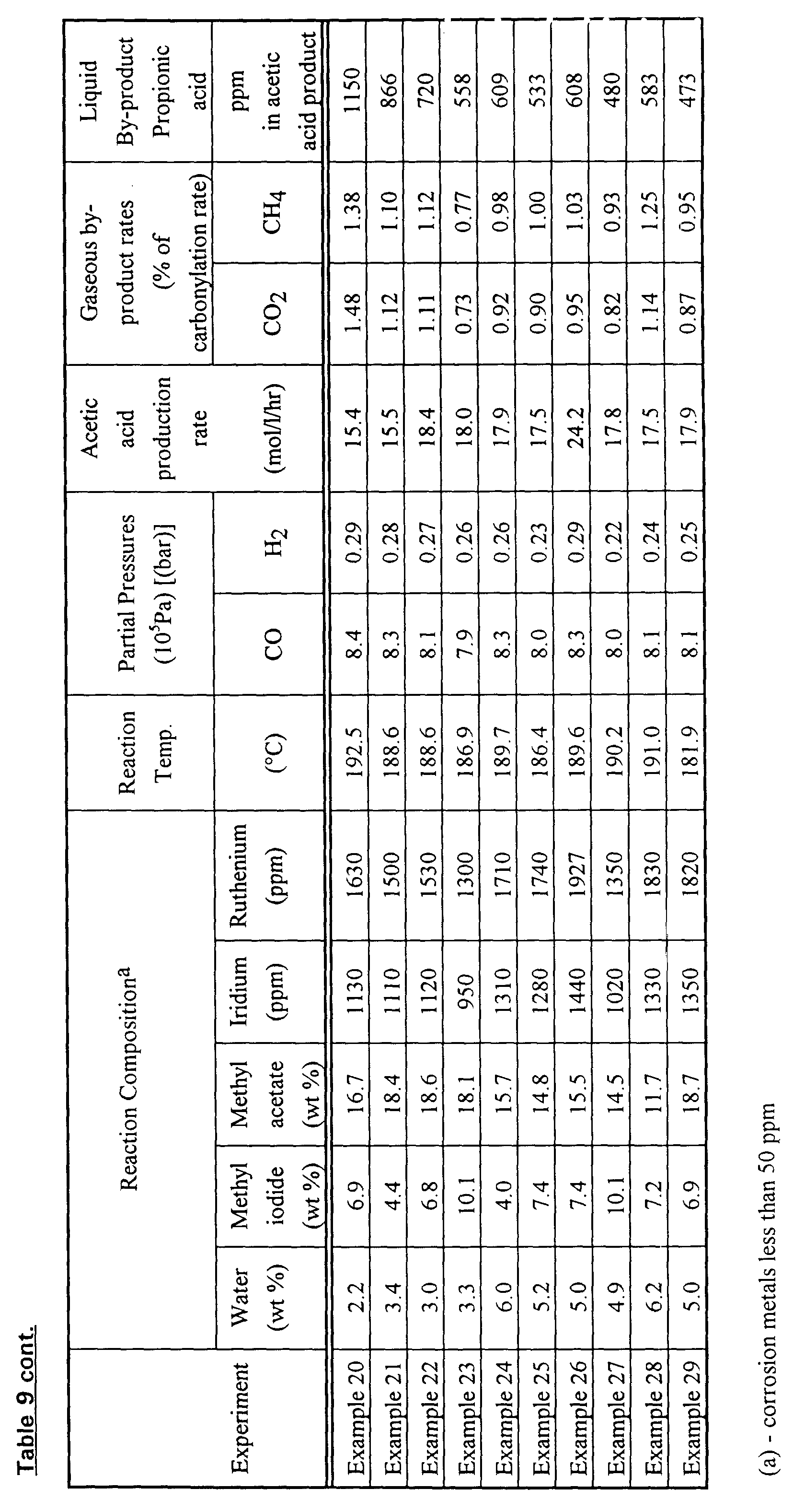

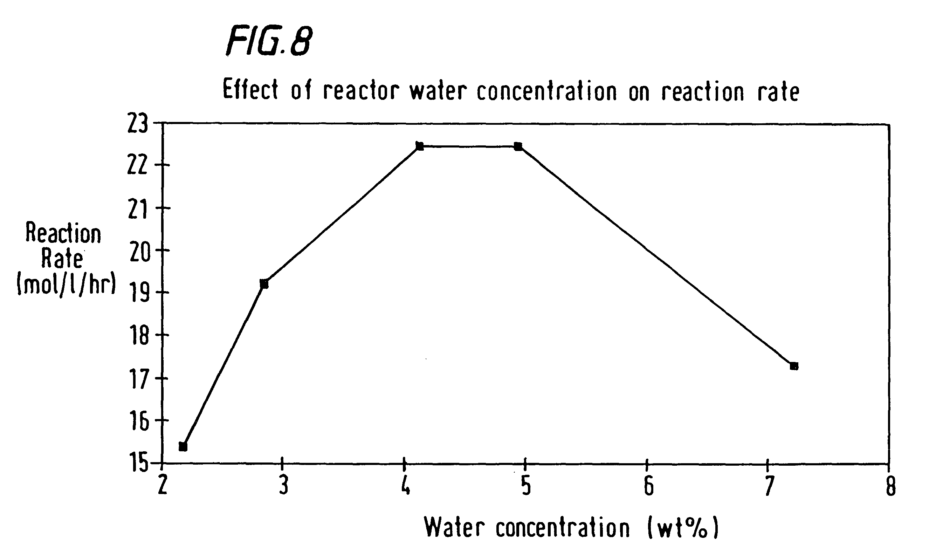

The results in Table 9 show the effect on carbonylation reaction rate and by-products of varying the water concentration from 7.1 % by weight to 2.2 % weight. From the data in Table 9 it can be seen that as the water concentration was reduced from above 6.5 % by weight to 6.5 % or less, the rate of acetic acid production reaction increased to a maximum (as in the batch examples), which under the particular conditions of Examples 17 to 20, was at about 4 to 5 % by weight. This is shown in graph form in Figure 8.

Examples 21 to 23 were reactions with a liquid reaction composition of about 18 % by weight methyl acetate, 4 to 10 % by weight methyl iodide co-catalyst and about 3 % by weight water. Examples 24 to 27 were reactions with a liquid reaction composition of about 15 % by weight methyl acetate and about 5 % by weight water. These two sets of examples show that as the methyl iodide co-catalyst concentration in the liquid reaction composition was increased, the temperature and/or catalyst concentration required to maintain a given reaction rate was reduced, together with a reduction of the by-product formation.

Examples 28 and 29 show the effect of varying water and methyl acetate concentrations at about 7 % by weight methyl iodide co-catalyst concentration in the liquid reaction composition. Thus, as the water concentration was reduced and the methyl acetate concentration was increased the temperature required to maintain the reaction rate was reduced and the by-product formation was reduced.

### Batch experiments using zinc promoter

Batch examples were performed as for Examples 1 to 12, but using zinc as a promoter in place of ruthenium. The charge compositions are set out in Table 10 and the reaction rate data in Table 11 below.

**Table 10**

| **Charge compositions for zinc promoted reactions in a 300 ml zirconium batch autoclave.** | | | | | | | |
|---|---|---|---|---|---|---|---|
| Example | Ref. | Methyl acetate (g) | Acetic acid (g) | Methyl iodide (g) | Water (g) | H₂IrCl₆ (g)^{a} | ZnI₂ (g) |
| 30 | 628 | 60.01 | 55.35 | 13.98 | 20.11 | 0.643 | 4.988 |
| 31 | 663 | 60.00 | 59.41 | 13.97 | 16.13 | 0.645 | 4.983 |
| 32 | 627 | 60.10 | 63.49 | 13.96 | 11.97 | 0.641 | 5.000 |

| | | | | | | | |
|---|---|---|---|---|---|---|---|
| (a) Weight expressed as pure H₂IrCl₆. | | | | | | | |

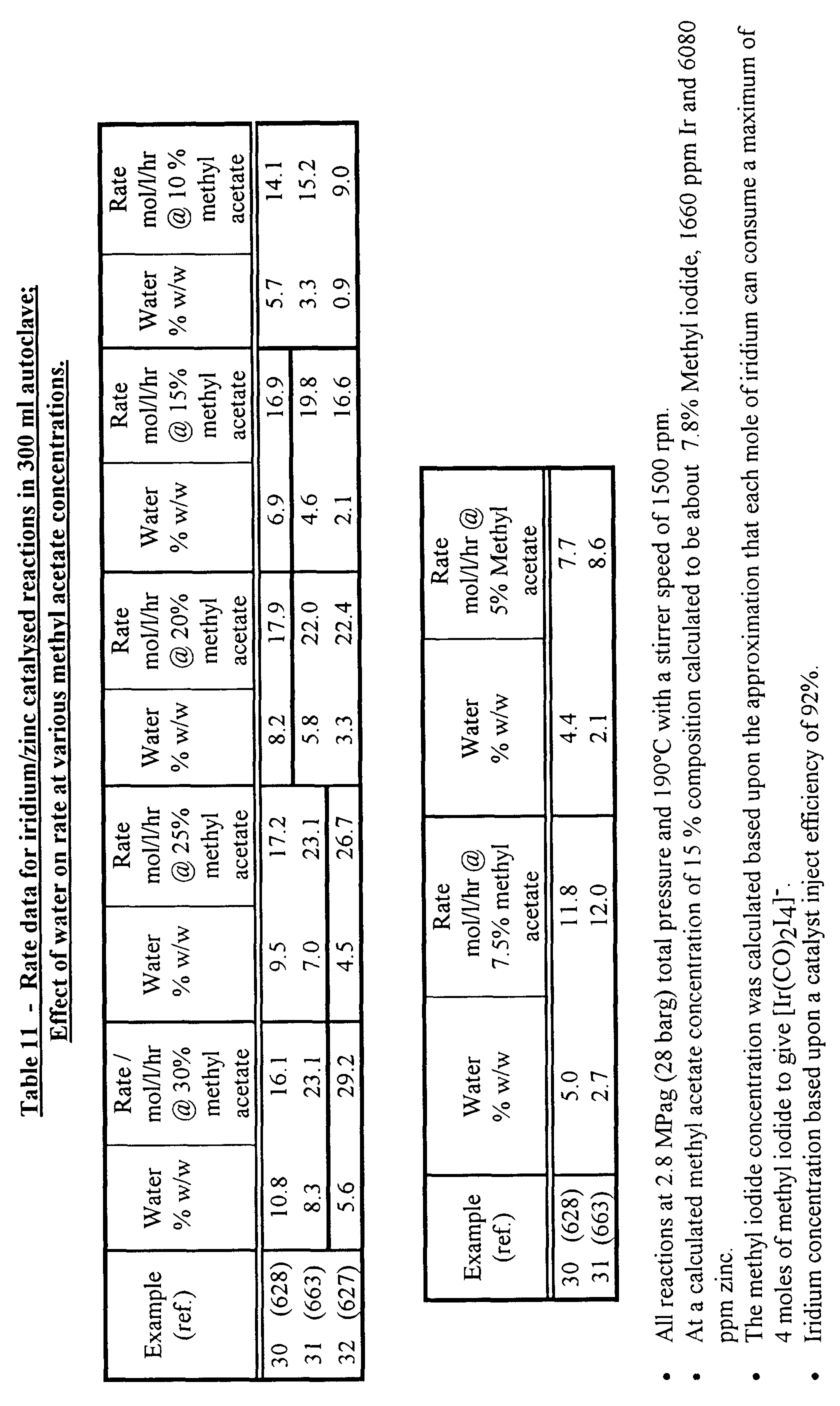

The data in Table 11 show that as the water concentration was reduced, the rate of carbonylation reaction increased and passed through a maximum at a water concentration no greater than 6.5 % by weight water.

## Claims

1. A process for the production of acetic acid comprising (1) continuously feeding methanol and/or a reactive derivative thereof and carbon monoxide to a carbonylation reactor which contains a liquid reaction composition comprising an iridium carbonylation catalyst, methyl iodide co-catalyst, a finite concentration of water, acetic acid, methyl acetate and at least one promoter; (2) contacting the methanol and/or reactive derivative thereof with the carbon monoxide in the liquid reaction composition to produce acetic acid; and (3) recovering acetic acid from the liquid reaction composition **characterised in that** there is continuously maintained in the liquid reaction composition throughout the course of the reaction (a) water at a concentration of 1 to 6.5 % by weight, (b) methyl acetate at a concentration in the range 5 to 30 % by weight and (c) methyl iodide at a concentration in the range 5 to 16 % by weight and further **in that** the water concentration is that which gives the maximum carbonylation rate at the defined methyl acetate and methyl iodide concentrations.

2. A process as claimed in claim 1 in which there is continuously maintained in the liquid reaction composition throughout the course of the reaction, methyl acetate at a concentration in the range 5 to 25 % by weight.

3. A process as claimed in any one of the preceding claims in which the at least one promoter is selected from the group consisting of ruthenium, osmium, rhenium, cadmium, mercury, zinc, gallium, indium and tungsten.

4. A process as claimed claim 3 wherein the promoter is either ruthenium or osmium.

5. A process as claimed in either claim 3 or claim 4 in which the molar ratio of promoter : iridium is [0.5 to 15] : 1.

6. A process as claimed in claim 1 in which there is continuously maintained in the liquid reaction composition throughout the course of the reaction about 5 % by weight water, about 7 % by weight methyl iodide, about 15 % methyl acetate, iridium catalyst at a concentration in the range 400 to 3000 ppm and ruthenium promoter at a concentration in the range 400 to 4000 ppm.

7. A process as claimed in any one of the preceding claims wherein the partial pressure of hydrogen in the carbonylation reactor is less than 0.1 MPa (1 bar).

8. A process as claimed in claim 7 wherein the partial pressure of hydrogen is less than 0.05 MPa (0.5 bar).

9. A process as claimed in claim 7 wherein the partial pressure of hydrogen is less than 0.03 MPa (0.3 bar).

10. A process as claimed in any one of the preceding claims wherein the amount of hydrogen in the carbon monoxide reactant is less than 1 mol %.

11. A process as claimed in claim 10 wherein the amount of hydrogen in the carbon monoxide reactant is less than 0.5 mol %.

12. A process as claimed in claim 10 wherein the amount of hydrogen in the carbon monoxide reactant is less than 0.3 mol %.

## Patentansprüche

1. Verfahren zur Herstellung von Essigsäure, umfassend (1) kontinuierliches Zuführen von Methanol und/oder einem reaktiven Derivat davon und Kohlenmonoxid zu einem Carbonylierungsreaktor, der eine flüssige Reaktionszusammensetzung, umfassend einen Iridiumcarbonylierungskatalysator, Methyljodid-Cokatalysator, eine endliche Konzentration Wasser, Essigsäure, Essigsäuremethylester und mindestens einen Promotor, enthält; (2) Inkontaktbringen des Methanols und/oder eines reaktiven Derivats davon mit dem Kohlenmonoxid in der flüssigen Reaktionszusammensetzung, unter Herstellung von Essigsäure; und (3) Gewinnen von Essigsäure aus der flüssigen Reaktionszusammensetzung, **dadurch gekennzeichnet, dass** in der flüssigen Reaktionszusammensetzung über den Verlauf der Reaktion stets (a) Wasser bei einer Konzentration von 1 bis 6,5 Gewichtsprozent, (b) Essigsäuremethylester bei einer Konzentration im Bereich von 5 bis 30 Gewichtsprozent und (c) Methyljodid bei einer Konzentration im Bereich von 5 bis 16 Gewichtsprozent gehalten wird, und weiterhin dadurch, dass die Wasserkonzentration jene ist, die die maximale Carbonylierungsgeschwindigkeit bei den festgelegten Essigsäuremethylester- und Methyljodidkonzentrationen ergibt.

2. Verfahren nach Anspruch 1, wobei in der flüssigen Reaktionszusammensetzung über den Verlauf der Reaktion Essigsäuremethylester stets bei einer Konzentration im Bereich von 5 bis 25 Gewichtsprozent gehalten wird.

3. Verfahren nach einem der vorangehenden Ansprüche, wobei der mindestens eine Promotor aus der Gruppe, bestehend aus Ruthenium, Osmium, Rhenium, Cadmium, Quecksilber, Zink, Gallium, Indium und Wolfram, ausgewählt ist.

4. Verfahren nach Anspruch 3, wobei der Promotor Ruthenium oder Osmium ist.

5. Verfahren nach entweder Anspruch 3 oder Anspruch 4, wobei das Molverhältnis von Promotor:Iridium [0,5 bis 15]:1 ist.

6. Verfahren nach Anspruch 1, wobei in der flüssigen Reaktionszusammensetzung über den Reaktionsverlauf stets etwa 5 Gewichtsprozent Wasser, etwa 7 Gewichtsprozent Methyljodid, etwa 15% Essigsäuremethylester, Iridiumkatalysator bei einer Konzentration im Bereich 400 bis 3000 ppm und Rutheniumpromotor bei einer Konzentration im Bereich von 400 bis 4000 ppm gehalten werden.

7. Verfahren nach einem der vorangehenden Ansprüche, wobei der Wasserstoffpartialdruck in dem Carbonylierungsreaktor weniger als 0,1 MPa (1 bar) ist.

8. Verfahren nach Anspruch 7, wobei der Wasserstoffpartialdruck weniger als 0,05 MPa (0,5 bar) ist.

9. Verfahren nach Anspruch 7, wobei der Wasserstoffpartialdruck weniger als 0,03 MPa (0,3 bar) ist.

10. Verfahren nach einem der vorangehenden Ansprüche, wobei die Menge an Wasserstoff in dem Kohlenmonoxidreaktanten weniger als 1 Mol% ist.

11. Verfahren nach Anspruch 10, wobei die Menge an Wasserstoff in dem Kohlenmonoxidreaktanten weniger als 0,5 Mol% ist.

12. Verfahren nach Anspruch 10, wobei die Menge an Wasserstoff in dem Kohlenmonoxidreaktanten weniger als 0,3 Mol% ist.

## Revendications

1. Procédé pour la production d'acide acétique, comprenant les étapes consistant (1) à introduire de manière continue du méthanol et/ou un de ses dérivés réactifs et du monoxyde de carbone dans un réacteur de carbonylation qui contient une composition réactionnelle liquide comprenant un catalyseur de carbonylation à l'iridium, un co-catalyseur consistant en iodure de méthyle, une concentration finie d'eau, de l'acide acétique, de l'acétate de méthyle et au moins un promoteur ; (2) à mettre en contact le méthanol et/ou son dérivé réactif avec le monoxyde de carbone dans la composition réactionnelle liquide pour produire de l'acide acétique ; et (3) à recueillir l'acide acétique à partir de la composition réactionnelle liquide, **caractérisé en ce que** sont maintenus de manière continue dans la composition réactionnelle liquide pendant toute la réaction (a) de l'eau à une concentration de 1 à 6,5 % en poids, (b) de l'acétate de méthyle à une concentration de 5 à 30 % en poids et (c) de l'iodure de méthyle à une concentration de 5 à 16 % en poids, et, en outre, **en ce que** la concentration d'eau est la concentration qui engendre la vitesse de carbonylation maximale aux concentrations définies en acétate de méthyle et iodure de méthyle.

2. Procédé suivant la revendication 1, dans lequel est maintenu de manière continue dans la composition réactionnelle liquide, pendant toute la réaction, de l'acétate de méthyle à une concentration comprise dans l'intervalle de 5 à 25 % en poids.

3. Procédé suivant l'une quelconque des revendications précédentes, dans lequel ledit au moins un promoteur est choisi dans le groupe consistant en ruthénium, osmium, rhénium, cadmium, mercure, zinc, gallium, indium et tungstène.

4. Procédé suivant la revendication 3, dans lequel le promoteur consiste en ruthénium ou osmium.

5. Procédé suivant la revendication 3 ou la revendication 4, dans lequel le rapport molaire promoteur : iridium est égal à [0,5 à 15] : 1.

6. Procédé suivant la revendication 1, dans lequel sont maintenus de manière continue dans la composition réactionnelle liquide, pendant toute la réaction, environ 5 % en poids d'eau, environ 7 % en poids d'iodure de méthyle, environ 15 % d'acétate de méthyle, le catalyseur à l'iridium à une concentration comprise dans l'intervalle de 400 à 3000 ppm et le promoteur au ruthénium à une concentration comprise dans l'intervalle de 400 à 4000 ppm.

7. Procédé suivant l'une quelconque des revendications précédentes, dans lequel la pression partielle d'hydrogène dans le réacteur de carbonylation est inférieure à 0,1 MPa (1 bar).

8. Procédé suivant la revendication 7, dans lequel la pression partielle d'hydrogène est inférieure à 0,05 MPa (0,5 bar).

9. Procédé suivant la revendication 7, dans lequel la pression partielle d'hydrogène est inférieure à 0,03 MPa (0,3 bar).

10. Procédé suivant l'une quelconque des revendications précédentes, dans lequel la quantité d'hydrogène dans le corps réactionnel consistant en monoxyde de carbone est inférieure à 1 % en moles.

11. Procédé suivant la revendication 10, dans lequel la quantité d'hydrogène dans le corps réactionnel consistant en monoxyde de carbone est inférieure à 0,5 % en moles.

12. Procédé suivant la revendication 10, dans lequel la quantité d'hydrogène dans le corps réactionnel consistant en monoxyde de carbone est inférieure à 0,3 % en moles.
